(19) Europäisches Patentamt
European Patent Office
Office européen des brevets

(11) Publication number: **0 090 360**
**B1**

(12) **EUROPEAN PATENT SPECIFICATION**

(45) Date of publication of patent specification: **18.05.88**

(21) Application number: **83102950.9**

(22) Date of filing: **24.03.83**

(51) Int. Cl.$^4$: **C 07 D 471/04,**
C 07 D 487/04,
C 07 D 498/04,
C 07 D 513/04,
C 07 D 497/04,
C 07 D 495/04,
C 07 D 493/04, A 61 K 31/47
// (C07D498/04, 263:00,
221:00)

# (54) New quinoline and related compounds useful as anti-allergy agents.

(30) Priority: **29.03.82 US 362712**

(43) Date of publication of application:
**05.10.83 Bulletin 83/40**

(45) Publication of the grant of the patent:
**18.05.88 Bulletin 88/20**

(84) Designated Contracting States:
**AT BE CH DE FR GB IT LI NL SE**

(56) References cited:

**JOURNAL OF APPLIED CHEMISTRY AND BIOTECHNOLOGY, vol. 27, 1977, pages 33-38, Oxford, GB A.M. OSMAN et al.: "Heterocyclic compounds. X. Synthesis of some oxazoloquinoline derivatives"**

**JOURNAL OF CHEMICAL TECHNOLOGY AND BIOTECHNOLOGY, vol. 32, March 1982, pages 485-488, Society of Chemical Industry, Oxford, GB A.S. HAMMAM et al.: "Synthesis of some new oxazoloquinolines and stilbyloxazoloquinolines"**

(73) Proprietor: **RORER INTERNATIONAL (OVERSEAS) INC.**
**1209 Orange Street**
**Wilmington Delaware (US)**

(72) Inventor: **Musser, John H.**
**Rd. 1, 15 Steiner Drive**
**Mahopac New York (US)**
Inventor: **Brown, Richard E.**
**16 Ridge Drive**
**East Hanover New Jersey (US)**

(74) Representative: **Patentanwälte Grünecker, Kinkeldey, Stockmair & Partner**
**Maximilianstrasse 58**
**D-8000 München 22 (DE)**

**EP 0 090 360 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to the general formula 1

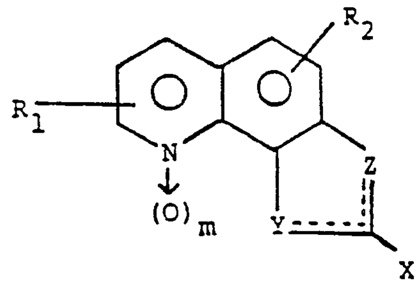

(I)

and salts thereof, wherein

$R_1$ is hydrogen, $C_1$—$C_5$ alkyl, halogen, trifluoromethyl, amino, benzyl, cyano or nitro,

$R_2$ is hydrogen, $C_1$—$C_5$ alkyl, halogen, trifluoromethyl, amino, $C_1$—$C_5$ alkylamino, $C_1$—$C_5$ acylamino, cyano, nitro or carboxyl,

m is 0 or 1,

Y is oxygen, sulfur, nitrogen or $NR_3$ wherein $R_3$ is hydrogen, $C_1$—$C_5$ alkyl, $C_1$—$C_5$ aminoalkyl or carboxyalkyl with up to 5 carbon atoms,

Z is oxygen, sulfur or nitrogen,

X is cyano, formyloximino, tetrazolyl or $(CH_2)_a$-CO—O—$R_4$

wherein a is 0 or 1 and $R_4$ is hydrogen, $C_1$—$C_{12}$ alkyl or $C_1$—$C_{12}$ alkyl substituted by a $C_1$—$C_5$ alkoxy or amino group, and the compounds 5-(N,N-dimethylamino)-2-carbomethoxy-1,3-oxazolo-[4,5-h]quinoline, 5-cyano-1,3-oxazolo-[4,5-h]-quinoline-2-propanoic acid methyl ester, and 2-carboxy-5-nitro-1,3-oxazolo-[4,5-h]-quinoline diethylaminoethyl ester.

These compounds have utility as medicinals, especially for treatment of asthma, and/or as intermediates in the preparation of compounds useful as medicinals.

In the preferred compounds, Z is nitrogen, $R_3$ is hydrogen, alkyl, aminoalkyl, or carboxyalkyl with up to 5 carbon atoms and m is 0.

It is preferable that when Y is oxygen, that Z be nitrogen; that when Z is sulfur, that Y be nitrogen; and that when Z is nitrogen, that Y be oxygen, sulfur or $R_3N$.

In the most preferred compounds, Z is nitrogen and Y is oxygen. In slightly less preferred compounds, Z is oxygen and Y is nitrogen.

When X includes an alkyl group, it contains 1 to 5 carbon atoms.

The preferred X groups are those including carboxy groups and, more preferably, carboxy groups directly attached to the ring. These groups include those having the general formula

$$(CH_2)a\text{—}\overset{\displaystyle O}{\overset{\|}{C}}\text{—}OR_4$$

wherein a is 0 or 1, preferably 0, and $R_5$ is H or an alkyl group, having 1—12 carbon atoms, preferably having 1—5 carbon atoms, or a $C_1$—$C_{12}$ alkyl group substituted with a $C_1$—$C_5$ alkoxy or amino group. $R_4$ can also be a metal or organic cation, preferably an alkali metal cation.

The compounds in which X is formyloximino or cyano are preferred as intermediates in a process for making the preferred carboxy compounds of this invention.

If $R_1$ is ethyl, it is preferably methyl or ethyl; if $R_1$ is halogen, it is preferably chlorine or bromine. It is preferred that $R_1$ is hydrogen. If $R_2$ is halogen, it is preferably chlorine. It is preferred that $R_2$ be at the 5 position.

The quinoline compounds of the present invention can be prepared by reaction of the following compounds under condensation conditions to form the desired heterocyclic ring:

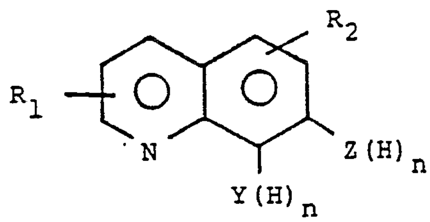

\+ X - R

III IV

wherein $R_1$, X, Y, Z and $R_2$ are as hereinbefore defined;

n = 1 or 2 and

R = a di- or trifunctional group capable of condensating with $Y(H)_n$ and $Z(H)_n$ to form the indicated heterocyclic ring, e.g., trihalomethyl, trialkyoxmethyl, or formyloximino.

A typical procedure for preparing the present new quinoline compounds where Z is N and Y is 0, S or $R_3N$ follows:

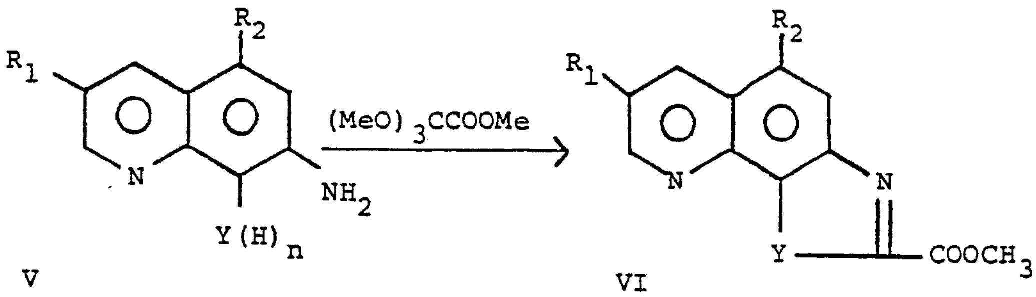


V VI

In this illustration, X is carbomethoxy but may be any of the groups representative of X.

A particularly preferred procedure for production of the present new compounds, especially the preferred compounds in which a carboxy group attached to the ring involves condensation of

R—C=NOH
  |
  H

with a selected 7-amino-8-hydroxyquinoline to form the corresponding 2-formyloximino-1,3-oxazolo[4,5-h] quinoline from which the corresponding cyano compound can be prepared by dehydration of the oximino compound and the cyano compound then converted to carboxy or carbalkoxy by known hydrolysis or alcoholysis reaction. The condensation with the oxime is usually carried out in a reaction solvent, preferably but not essentially, in the presence of a base catalyst such as alkali metal salts of organic carboxylic acids such as salts of acetic acid, e.g., sodium acetate. The use of temperatures higher than room temperature merely shortens the requisite reaction time. Temperatures from 0°C up to 150°C can be used.

Compounds in which m=1, i.e., the N-oxides, can be formed by reaction of corresponding compounds in which m=0 with peroxide or equivalent peracids. Thus, reaction is effected with hydrogen peroxide, perbenzoic acid, peracetic acid, and the other peroxides commonly used for this purpose. Generally, the N-oxide formation is carried out at room temperature or lower, to as low as 0°C, for example. For conveneience, the starting compound may be dissolved in a suitable reaction solvent. Although only equivalent amounts of peroxide are required, usually excess is used to assure complete reaction.

Solvents employed in the present preparative processes may be any of those commonly used in organic preparations such as dioxane, tetrahydrofyran, dimethyl acetamide, dimethyl formamide and similar solvents. Solvents are not always necessary, however, since the condensation of compounds of formula III with those of formula IV can be carried out without solvent by mere mixing of the reactants, preferably with use of reaction temperature above room temperature, up to above 150°C and preferably from about 50° to about 125°C.

The invention will be more fully illustrated in the examples which follow.

## Example 1

A. *5-Chloro-7-nitro-8-hydroxyquinoline*

To a solution of 5-chloro-8-hydroquinoline (90.0 g, 0.5 mol) in sulfuric acid (500 ml) at 0°C was added 90% nitric acid (0.6 mol) at such a rate that the temperature did not exceed 2°C. The clear solution was stirred for one hour at 0°C, and then allowed to slowly warm to room temperature. The mixture was poured into ice (2 liter) and stirred overnight. The yellow precipitate was filtered, washed with water and dried. The yellow cake was crystallized from methylethylketone giving 85.0 g (76% yield) of solid. M.P. 192—194°C.

B. *5-Chloro-7-amino-8-hydroxyquinoline*

To a Suspension of 5-chloro-7-nitro-8-hydroxyquinoline (85.0 g, 0.38 mol) in a 1:1 mixture of methanol and water (2.5 liter) was added sodium dithionite (340 g, 2.0 mol). The reaction which is slightly exothermic was stirred overnight under nitrogen. The yellow solid was filtered, washed with water and crystallized from ethanol giving 52 g (70% yield) of solid. M.P. 162-164°C.

C. *2-Carbomethoxy-5-chloro-1,3-oxazolo-[4,5-h] quinoline*

A mixture of 5-chloro-7-8-hydroaminoquinoline (6 g, 30.8 mol) and methyl trimethyoxyacetate (16.3 g, 123.2 mol) was heated at 100°C overnight. The reaction was cooled in an ice bath and filtered. The precipitate was dissolved in acetone (250 ml). The resulting solution was treated with charcoal at reflux,

filtered through a pad of silica gel and celite and partially concentrated. Crystals formed which were filtered and dried giving 4.1 g (51% yield) of solid. M.P. 217—218°C.

Example 2

*2-Formyloximino-5-chloro-1,3-oxazolo-[4,5h] quinoline*

A solution of chloral hydrate (1.7 g, 10 mmol) in water (10 ml) containing an equivalent amount of hydroxylamino to form the oxime was heated at 60°C for three hours. Then a solution of 5-chloro-7-nitro-8-hydroxyquinoline (0.9 g, 4.6 mmol) in DMF (10 ml) was added. To this mixture was added sodium acetate (3.2 g, 40 mmol) portionwise over a two hour period. The reaction was heated at 60°C for an additional hour. The solvent was removed in vacuo. The remaining material was triturated with water, filtered and dried. The solid was dissolved in acetone, treated with charcoal and filtered through a pad of celite gel. The solvent was removed giving the desired product. M.P. 220°C — dec.

Example 3

*2-Cyano-5-chloro-1,3-oxazolo-[4,5-h] quinoline*

A suspension of 2-formyloximino-5-chloro-1,3-oxazolo [4,5-h] quinoline (0.7 g, 2.8 mmol) in toluene (300 ml) was treated with thionyl chloride (0.3 ml) and refluxed for one hour. The reaction was filtered and concentrated. The remaining material was dissolved in acetone and filtered through a pad of silica gel and celite. The solvent was removed giving the desired product. M.P. 214—215°C.

Example 4

*2-Carbomethoxy-5-chloro-1,3-oxazolo-[4,5-h] quinoline*

A solution of 5 g of 2-cyano-5-chloro-1,3-oxazolo-4,5-h] quinoline in 100 ml of methanol was cooled to 0°C and dry HCl was bubbled through the solution for 1 hour. The solution was then maintained at 0°C for 24 hours, then allowed to warm up to room temperature. The solvent was removed in vacuo and the residue was dissolved in acetone and filtered through a pad of silica gel and celite. The solvent was removed to give the desired product. M.P. 214—215°C.

Examples 5 and 6

In like manner as above using 7-amino-5-chloro-8-hydroxyquinoline and the appropriate ortho esters or imidates, the following compounds were prepared:

5. 5-Chloro-1,3-oxazolo-[4,5-h] quinoline 2-acetic acid, ethyl ester. M.P. 118—121°C.
6. 2-Carboethoxy-5-chloro-1,3-oxazolo-[4,5-h] quinoline M.P. 150—152°C.

Examples 7 to 15

The following compounds can be made using the above procedure and the appropriate aminoquinolines and imidates or ortho esters:

7. 8-methyl-2-carbomethoxy-1,3-oxazolo-[4,5-h] quinoline.
8. 5-(N,N-dimethylamino)-2-carboxymethoxy-1,3-oxazolo-[4,5-h] quinoline.
9. 2-carboethoxy-1,3-oxazolo-[4,5-h] quinoline.
10. 8-trifluoromethyl-2-carbomethyl-1,3-oxazolo-[4,5-h] quinoline.
11. 5-cyano-1,3-oxazolo-[4,5-h] quinoline-2-propanoic acid, methyl ester.
12. 5-bromo-2-(5-tetrazolyl)-1,3-oxazolo-[4,5-h] quinoline.
13. 5-methyl-2-carbopentoxy-1,3-oxazolo-[4,5-h] quinoline.
14. 2-carboxy-5-trifluoromethyl-1,3-oxazolo-[4,5-h] quinoline ethoxyethyl ester.
15. 2-carboxy-5-nitro-1,3-oxazolo-[4,5-h] quinoline diethylaminoethyl ester.

Example 16

*5-chloro-1,3-oxazolo-[4,5-h] quinoline-2-carboxylate, sodium salt*

A suspension of 2-carboethoxy-5-chloro-1,3-oxazolo-[4,5-h] quinoline (1.5 g) in water (100 ml) was treated with 16.3 ml of 1N NaOH. After 10 minutes, the aqueous phase was extracted with chloroform. The aqueous phase was then treated with saturated ammonium chloride causing a white precipitate for form. The filtrate was concentrated in vacuo giving 0.9 g of solid, M.P. 212—216°C which was suspended in water (50 ml), treated with one equivalent of sodium hydroxide, and lyophilized to give 0.9 g of product. M.P. 200°C — dec.

Example 17

In like manner as above using the approprate base, the following salt was prepared:

Tris (hydroxymethyl) ammonium methane 5-chloro-1,3-oxazolo-[4,5-h] quinoline-2-carboxylate salt. M.P. 90°C — dec.

Example 18

*2-(5-tetrazolo-5-chloro-1,3-oxazolo-[4,5-h] quinoline*

A mixture of 5 g of 2-cyano-5-chloro-1,3-oxazolo-[4,5-h] quinoline and 1.0 g of sodium azide and 2 g of ammonium chloride in 100 ml of DMF are heated for 3 hours at 120°C. The reaction mixture is poured into water acidified with dilute HCl, and the product is filtered and recrystallized.

Example 19

Other compounds within the current invention which can be prepared include the following in which $R_1$, $R_2$ and X can be disclosed above.

(A) Oxazolo-[5,4-h] quinolines as, for example, 2-carbomethoxy-1,3-oxazolo-[4,5-h] quinoline

(B) Imidazo-[4,5-h] quinolines as, for example, 2-carbomethoxy-1,3-oxazolo-[4,5-h] quinoline

(C) Thiazolo-[4,5-h] quinoline as, for example, 2-carbomethoxy-1,3-thiazolo-[4,5-h] quinoline, and 2-carbomethoxy-1,3-thiazolo-[4,5-h] quinoline-N-oxide

(D) Thiazolo--[4,5-h] quinolines as, for example, 2-carbomethoxy-1,3-thiazolo-[4,5-h] quinoline

(E) Oxazolo-[4,5-h] quinolines as, for example, 2-carbomethoxy-5-chloro-1,3-oxazolo-[4,5-h] quinoline-N-oxide.

The compounds of this invention are useful as anti-allergy agents as determined by testing in the following procedures recognized to be cognent *in vitro* and *in vivo* models of human allergic disease.

A. The PCA Test

The compounds of this invention have potent activity in inhibiting the formation of a wheal when screened according to the Rat Passive Cutaneous Anaphylaxis (PCA) Screen as described by I. Mota, Life Sciences, *7,* 465 (1963) and Z. Ovary, et al. Proceedings of Society of Experimental Biology and Medicine, *81,* 584 (1952).

B. The RMC Test

In addition, the compound of this invention have potent activity as inhibitors of histamine release from passively sensitized Rat Mast Cells according to the procedure described by E. Kusner, et al., Journal of Pharmacology and Experimental Therapeutics, *184,* 41 (1973).

When screened according to the above procedures, the compounds of this invention showed potent anti-allergic activity and, as such, are useful in the treatment of conditions such as asthma. In the RMC test $I_{50}$ values of from 1 to 100 μm were found; in the PCA test, $ED_{50}$ values of from 1 to 50 mg/kg were found. By way of illustration, the compound of example 1C had an $I_{50}$ value of 0.3 μm in the RMC test and $E_{50}$ value of 0.5 mg/kg and 1.0 mg/kg when administered by the intraperitoneal route and oral routes respectively in PCA test. The compound of example 6 had an $I_{50}$ value of 0.1μm in the RMC test and 59% inhibition of wheal formation at 10 mg/kg in the PCA test on intraperitoneal administration. The compound of example 16 had an $I_{50}$ of 0.1 m in the RMC test and 51% inhibition of wheal formation at 10 mg/kg in the PCA test on intraperitoneal administration.

**Claims for the Contracting States: BE CH DE FR GB IT LI NL SE**

1. A quinoline compound of the general formula 1

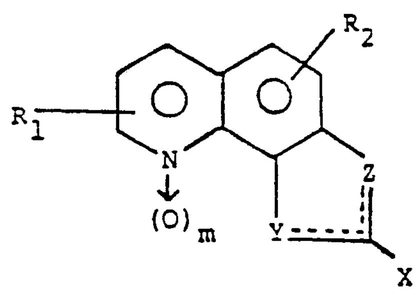

(I)

and salts thereof, wherein

$R_1$ is hydrogen, $C_1$—$C_5$ alkyl, halogen, trifluoromethyl, amino, benzyl, cyano or nitro,

$R_2$ is hydrogen, $C_1$—$C_5$ alkyl, halogen, trifluoromethyl, amino, $C_1$—$C_5$ alkylamino, $C_1$—$C_5$ acylamino, cyano, nitro or carboxyl,

m is 0 or 1,

Y is oxygen, sulfur, nitrogen or $NR_3$ wherein $R_3$ is hydrogen, $C_1$—$C_5$ alkyl, $C_1$—$C_5$ aminoalkyl or carboxyalkyl with up to 5 carbon atoms,

Z is oxygen, sulfur or nitrogen,

X is cyano, formyloximino, tetrazolyl or $(CH_2)_a$-CO—O—$R_5$

wherein a is 0 or 1 and $R_4$ is hydrogen, $C_1$—$C_{12}$ alkyl or $C_1$—$C_{12}$ alkyl substituted by a $C_1$—$C_5$ alkoxy or amino group, and the compounds 5-(N,N-dimethylamino)-2-carbomethoxy-1,3-oxazolo-[4,5-h]quinoline, 5-cyano-1,3-oxazolo-[4,5-h]-quinoline-2-propanoic acid methyl ester, and 2-carboxy-5-nitro-1,3-oxozolo-[4,5-h]-quinoline diethylaminoethyl ester.

2. A compound as in claim 1 wherein m is 0.

3. A compound as in claim 1 or 2 wherein Z is oxygen and Y is nitrogen.

4. A compound as in claim 1 or 2 wherein Z is sulphur and Y is nitrogen.

5. A compound as in claim 1 or 2 wherein Z is nitrogen and Y is sulfur.

6. A compound as in claim 1 or 2 wherein Z is nitrogen and Y is $R_3N$.

7. A process for preparing compounds of formula 1 and salts thereof according to any of claims 1 to 6 which comprises reacting under condensation conditions a compound of formula III

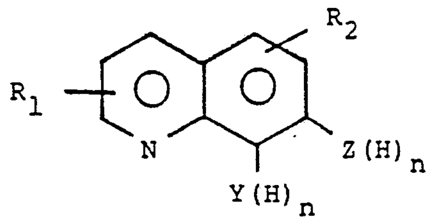

III

wherein $R_1$, $R_2$, Y and Z are as defined in claim 1, and n is 1 or 2 with a compound of formula IV

$$X—R \qquad (IV)$$

wherein X is as defined in claim 1, and R is a di- or trifunctional group capable of condensing with $Y(H)_n$ or $Z(H)_n$ to form a heterocyclic ring; and optionally forming the corresponding N-oxides of the compound so produced; and optionally forming salt of the compound so produced.

8. The process according to claim 7 wherein the product formed is 2-carboalkoxy compound which on saponification yields the corresponding 2-carboxy compound.

9. The process according to claim 7 wherein the product formed is a 2-formyloximino compound which on dehydration yields the corresponding 2-cyano compound.

10. The process according to claim 9, wherein the 2-cyano compound is hydrolyzed to the 2-carboxy compound.

11. The process according to claim 9 wherein the 2-cyano compound is alcoholized to the 2-carboalkoxy compound.

12. A pharmaceutical composition comprising a therapeutically effective amount of a compound according to any of claims 1 to 6.

**Claims for the Contracting State: AT**

1. A process of preparing a quinoline compound of the general formula 1

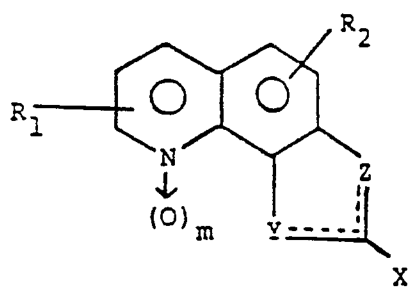

(I)

and salts thereof, wherein

$R_1$ is hydrogen, $C_1$—$C_5$ alkyl, halogen, trifluoromethyl, amino, benzyl, cyano or nitro,

$R_2$ is hydrogen, $C_1$—$C_5$ alkyl, halogen, trifluoromethyl, amino, $C_1$—$C_5$ alkylamino, $C_1$—$C_5$ acylamino, cyano, nitro or carboxyl,

m is 0 or 1,

Y is oxygen, sulfur, nitrogen or $NR_3$ wherein $R_3$ is hydrogen, $C_1$—$C_5$ alkyl, $C_1$—$C_5$ aminoalkyl or carboxyalkyl with up to 5 carbon atoms,

Z is oxygen, sulfur or nitrogen,

X is cyano, formyloximino, tetrazolyl or $(CH_2)_a$-CO—O—$R_5$

wherein a is 0 or 1 and $R_4$ is hydrogen, $C_1$—$C_{12}$ alkyl or $C_1$—$C_{12}$ alkyl substituted by a $C_1$—$C_5$ alkoxy or amino group, and the compounds 5-(N,N-dimethylamino)-2-carbomethoxy-1,3-oxazolo-[4,5-h]quinoline, 5-cyano-1,3-oxazolo-[4,5-h]-quinoline-2-propanoic acid methyl ester, and 2-carboxy-5-nitro-1,3-oxozolo-[4,5-h]-quinoline diethylaminoethyl ester,

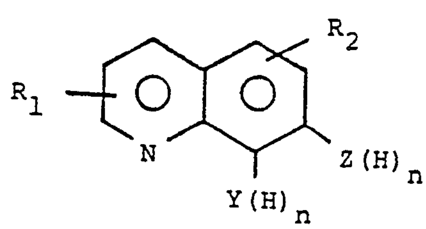

III

wherein $R_1$, $R_2$, Y and Z are as defined in claim 1, and n is 1 or 2 with a compound of formula IV

X—R (IV)

wherein X is as defined in claim 1, and R is a di- or trifunctional group capable of condensing with $Y(H)_n$ or $Z(H)_n$ to form a heterocyclic ring; and optionally forming the corresponding N-oxides of the compound so produced; and optionally forming salt of the compound so produced.

2. The process as in claim 1 wherein m is 0.

3. The process as in claim 1 or 2 wherein Z is oxygen and Y is nitrogen.

4. The process as in claim 1 or 2 wherein Z is sulphur and Y is nitrogen.

5. The process as in claim 1 or 2 wherein Z is nitrogen and Y is sulfur.

6. The process as in claim 1 or 2 wherein Z is nitrogen and Y is $R_3N$.

7. The process according to claim 1 wherein the product formed is a 2-carboalkoxy compound which on saponification yields the corresponding 2-carboxy compound.

8. The process according to claim 1 wherein the product formed is 2-carboalkoxy compound which on saponification yields the corresponding 2-carboxy compound.

9. The process according to claim 1 wherein the 2-cyano compound is hydrolized to the 2-carboxy compound.

10. The process according to claim 1, wherein the 2-cyano compound is alcoholized to the 2-carboalkoxy compound.

11. A process for preparing a pharmaceutical composition comprising formulating a therapeutically effective amount of a compound prepared according to any of claims 1 to 10.

**Patentansprüche für die Vertragsstaaten: BE CH DE FR GB IT LI NL SE**

1. Chinolinverbindung der allgemeinen Formel 1

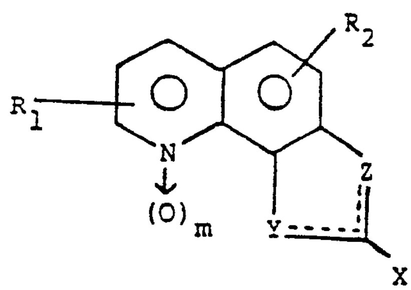

(I)

und Salze davon, worin

$R_1$ Wasserstoff, $C_1$—$C_5$-Alkyl, Halogen, Trifluoromethyl, Amino, Benzyl, Cyano oder Nitro ist,

$R_2$ Wasserstoff, $C_1$—$C_5$-Alkyl, Halogen, Trifluormethyl, Amino, $C_1$—$C_5$-Alkylamino, $C_1$—$C_5$-Acylamino, Cyano, nitro oder Carboxyl ist,

m 0 oder 1 ist,

Y Sauerstoff, Schwefel, Stickstoff oder $NR_3$ ist, worin $R_3$ Wasserstoff, $C_1$—$C_5$-Alkyl, $C_1$—$C_5$-Aminoalkyl oder Carboxyalkyl mit bis zu 5 Kohlenstoffatomen ist,

Z Sauerstoff, Schwefel oder Stickstoff ist,

X Cyano, Formyloximino, Tetrazolyl oder $(CH_2)_a$—CO—O—$OR_5$ ist, worin 0 oder 1 ist und $R_4$ Wasserstoff, $C_1$—$C_{12}$-Alkyl oder $C_1$—$C_{12}$-Alkyl, substituiert durch eine $C_1$—$C_5$-Alkoxy- oder Aminogruppe, ist, und die Verbindung 5-(N,N-Dimethylamino)-2-carbomethoxy-1,3-oxozolo-[4,5-h)chinolin, 5-Cyano-1,3-oxazolo-[4,5-h]chinolin-2-propansäure-methylester und 2-Carboxy-5-nitro-1,3-oxazolo-[4,5-h]chinolin-diethylaminoethylester.

2. Verbindung nach Anspruch 1, worin m 0 ist.

3. Verbindung nach Anspruch 1 oder 2, worin Z Sauerstoff und Y Stickstoff ist.

4. Verbindung nach Anspruch 1 oder 2, worin Z Schwefel und Y Stickstoff ist.

5. Verbindung nach Anspruch 1 oder 2, worin Z Stickstoff und Y Schwefel ist.

6. Verbindung nach Anspruch 1 oder 2, worin Z Stickstoff und Y $R_3N$ ist.

7. Verfahren zur Herstellung von Verbindungen der Formel I und Salzen davon nach einem der Ansprüche 1 bis 6, bei dem eine Verbindung der Formel III

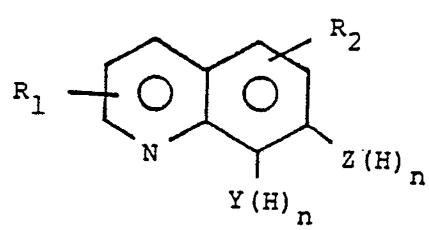

III

worin $R_1$, $R_2$ Y und Z wie in Anspruch 1 definiert sind und n 1 oder 2 ist unter Kondensationsbedingungen mit einer Verbindung der Formel IV

X—R (IV)

worin X wie in Anspruch 1 definiert ist und R eine di- oder trifunktionelle Gruppe ist, die mit $Y(H)_n$ oder $Z(H)_n$ zur Bildung eines heterocycloschen Rings kondensieren kann, umgesetzt, wird; und gegebenenfalls die entsprechenden N-Oxide der so hergesellten Verbindung gebildet werden; und gegebenenfalls Salze der so hergestellten Verbindung gebildet werden.

8. Verfahren nach Anspruch 7, worin das gebildete Produkt eine 2-Carboalkoxyverbindung ist, die bei Verseifung die entsprechende 2-Carboxyverbindung ergibt.

9. Verfahren nach Anspruch 7, worin das gebildete Produkt eine 2-Formyloximinoverbindung ist, die bei Dehydration die entsprechende 2-Cyanoverbindung ergibt.

10. Verfahren nach Anspruch 9, worin die 2-Cyanoverbindung zu der 2-Carboxyverbindung hydrolysiert wird.

11. Verfahren nach Anspruch 9, worin die 2-Cyanoverbindung zu der 2-Carboalkoxyverbindung alkoholisiert wird.

12. Pharmazeutische Zusammensetzung, umfassend eine therapeutisch wirksame Menge einer Verbindung nach einem der Ansprüche 1 bis 6.

**Patentansprüche für den Vertragsstaat: AT**

1. Verfahren zur Herstellung einer Chinolinverbindung der allgemeinen Formel 1

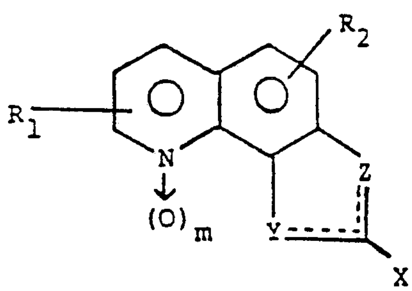

(I)

und Salze davon, worin

$R_1$ Wasserstoff, $C_1$—$C_5$-Alkyl, Halogen, Trifluromethyl, Amino, Benzyl, Cyano oder Nitro ist,

$R_2$ Wasserstoff, $C_1$—$C_5$-Alkyl, Halogen, Trifluormethyl, Amino, $C_1$—$C_5$-Alkylamino, $C_1$—$C_5$-Acylamino, Cyano, nitro oder Carboxyl ist,

m 0 oder 1 ist,

Y Sauerstoff, Schwefel, Stickstoff oder $NR_3$ ist, worin $R_3$ Wasserstoff, $C_1$—$C_5$-Alkyl, $C_1$—$C_5$-Aminoalkyl oder Carboxyalkyl mit bis zu 5 Kohlenstoffatomen ist,

Z Sauerstoff, Schwefel oder Stickstoff ist,

X Cyano, Formyloximino, Tetrazoyl oder $(CH_2)_a$—CO—O—$OR_5$ ist, worin 0 oder 1 ist und $R_4$ Wasserstoff, $C_1$—$C_{12}$-Alkyl oder $C_1$—$C_{12}$-Alkyl, substituiert durch eine $C_1C_5$-Alkoxy- oder Aminogruppe, ist, und die Verbindung 5-(N,N-Dimethylamino)-2-carbomethoxy-1,3-oxozolo-[4,5-h)chonilin, 5-Cyano-1,3-oxazolo-[4,5-h]chinolin-2-propansäure-methylester und 2-Carboxy-5-nitro-1,3-oxazolo-[4,5-h]chinolindiethylaminoethylester bei dem eine Verbindung der Formel III

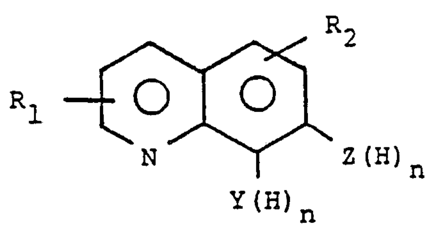

III

worin $R_1$, $R_2$ Y und Z wie in Anspruch 1 definiert sind und n 1 oder 2 ist unter Kondensationsbedingungen mit einer Verbindung der Formel IV

X—R (IV)

worin X wie in Anspruch 1 definiert ist und R eine zwei- oder dreifunktionelle Gruppe ist, die mit $Y(H)_n$

oder $Z(H)_n$ zur Bildung eines heterocyclischen Rings kondensieren kann, umgesetzt, wird; und gegebenenfalls die entsprechenden N-Oxide der so hergesellten Verbindung gebildet werden; und gegebenenfalls Salze der so hergestellten Verbindung gebildet werden.

2. Verfahren nach Anspruch 1, worin m 0 ist.

3. Verfahren nach Anspruch 1 oder 2, worin Z Sauerstoff und Y Stickstoff ist.

4. Verfahren nach Anspruch 1 oder 2, worin Z Schwefel und Y Stickstoff ist.

5. Verfahren nach Anspruch 1 oder 2, worin Z Stickstoff und Y Schwefel ist.

6. Verfahren nach Anspruch 1 oder 2, worin Z Stickstoff und Y $R_3N$ ist.

7. Verfahren nach Anspruch 1, worin das gebildete Produkt eine 2-Carboalkoxyverbindung ist, die bei Verseifung die entsprechende 2-Carboxyverbindung ergibt.

8. Verfahren nach Anspruch 1, worin das gebildete Produkt eine 2-Formyloximinoverbindung ist, die bei Dehydration die entsprechende 2-Cyanoverbindung ergibt.

9. Verfahren nach Anspruch 1, worin die 2-Cyanoverbindung zu der 2-Carboxyverbindung hydrolysiert wird.

10. Verfahren nach Anspruch 1, worin die 2-Cyanoverbindung zu der 2-Carboalkoxyverbindung alkoholisiert wird.

11. Verfahen zur Herstellung einer pharmazeutischen Zusammensetzung, umfassend die Formulierung einer therapeutisch wirksamen Menge einer Verbindung, hergestellt nach einem der Ansprüche 1 bis 10.

**Revendications pour les Etats contractants: BE CH DE FR GB IT LI NL SE**

1. Dérivé de la quinoléine représénte par la formule générale 1:

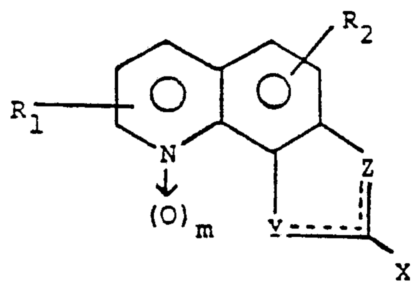

(I)

et ses sels où:

$R_1$ représente hydrogène, alkyle en $C_1$—$C_5$, halogène, trifluorométhyle, amino, benzyle, cyano ou nitro;

$R_2$ représente hydrogène, alkyle en $C_1$—$C_5$, halogène, trifluorométhyle, amino, alkylamino en $C_1$—$C_5$, acylamino en $C_1$—$C_5$, cyano, nitro ou carboxyle;

m vaut 0 ou 1;

Y représente oxygène, soufre, azote ou $NR_3$, où $R_3$ représente hydrogène, alkyle en $C_1$—$C_5$, aminoalkyle en $C_1$—$C_5$ ou carboxyalkyle ayant jusqu'à atomes de carbone;

Z représente oxygène, soufre ou azote;

X représente cyano, formyloximino, tétrazoyle ou $(CH_2)_a$—CO—O—$R_5$, où a vaut 0 ou 1 et $R_4$ représente hydrogène, alkyle en $C_1$—$C_{12}$ ou alkyle en $C_1$—$C_{12}$ substitué par un groupe alcoxy en $C_1$—$C_5$ ou amino, et les composés; (N,N-diméthylamino)-5-carbométhoxy-2-oxazolo-1,3-[4,5-h] quinoléine; ester méthylique de l'acide cyano-5-oxazolo-1,3-[4,5-h] quinoléine-propanoïque-2; et ester diéthylaminoéthylique de la carboxy-2-nitro-5-oxazolo-1,3-[4,5-h)quinoléine.

2. Composé selon la revendication 1, dans lequel m vaut 0.

3. Composé selon la revendication 1 ou 2, dans lequel Z représente oxygène et Y représente azote.

4. Composé selon la revendication 1 ou 2, dans lequel Z représente soufre et Y représente soufre et Y représente azote.

5. Composé selon la revendication 1 ou 2, dans lequel Z représente azote et Y représente soufre.

6. Composé selon la revendication 1 ou 2, dans lequel Z représente azote et Y représente $R_3N$.

7. Procedé préparation de composés représentes par la formule 1 et de leurs sels, tels qu'ils sont définis à l'une des revendications 1 à 6, qui consiste à faire réagir, dans des conditions de condensation, un composé de formule III:

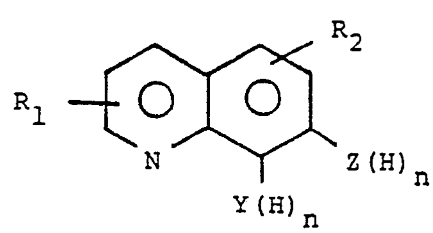

III

où:

R₁, R₂, Y et sont tels que définis à la revendications 1 et;

n vaut 1 ou 2, avec un composé de formule IV:

$$X—R \qquad (IV)$$

où

X est tel que défini à la revendication 1, et R représente un groupe di- ou trifonctionnel capable de se condenser avec $Y(H)_n$ ou $Z(H)_n$ afin de former un noyau hétérocyclique; et facultativement, à former les N-oxydes correspondants du composé ainsi produit; et faculativement, à former des sels du composé ainsi produit.

8. Procédé selon la revendication 7, dans lequel le produit formé est un composé carbalcoxy-2 qui, par saponification, donne le composé carboxy-2- correspondant.

9. Procédé selon la revendication 7, dans lequel le produit formé est un composé formyloximino-2 qui, par déhydration, donne le composé cyano-2-correspondant.

10. Procédé selon la revendication 9, dans lequel le composé cyano-2 est hydrolysé en le composé carboxy-2.

11. Procédé selon la revendication 9, dans lequel le composé cyano-2 est alcoolisé en le composé carbalcoxy-2.

12. Composition pharmaceutique comprenant une quantité thérapeutiquement efficace d'un composé tel que défini à l'une des revendications 1 à 6.

**Revendications pour L'etat contractant: AT**

1. Procédé de préparation d'un dérive de la quinoléine représenté par la formule genérale 1:

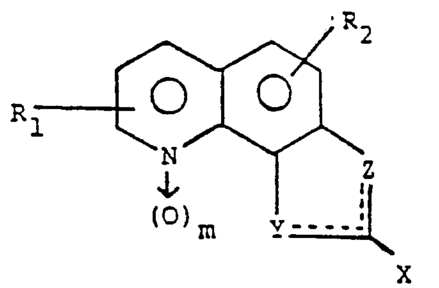

(I)

et de ses sels où:

$R_1$ représente hydrogène, alkyle en $C_1$—$C_5$, halogène, trifluorométhyle, amino, benzyle, cyano ou nitro;

$R_2$ représente hydrogène, alkyle en $C_1$—$C_5$, halogène, trifluorométhyle, amino, alkylamino en $C_1$—$C_5$, acylamino en $C_1$—$C_5$, cyano, nitro ou carboxyle;

m vaut 0 ou 1;

Y représente oxygène, soufre, azote ou $NR_3$, où $R_3$ représente hydrogène, alkyle en $C_1$—$C_5$, aminoalkyle en $C_1$—$C_5$, ou carboxyalkyle ayant jusqu'à atomes de carbone;

Z représente oxygène, soufre ou azote;

X représente cyano, formyloximino, tétrazoyle ou $(CH_2)_a$—CO—O—$R_4$, où a vaut 0 ou 1 et $R_4$ représente hydrogène, alkyle en $C_1$—$C_{12}$ ou alkyle en $C_1$—$C_{12}$ substitué par un groupe alcoxy en $C_1$—$C_5$ ou amino, et les composés; (N,N-diméthylamino)-5-carbométhoxy-2-oxazolo-1,3-[4,5-h] quinoléine; ester méthylique de l'acide cyano-5-oxazolo-1,3-[4,5-h] quinoléine-propanoïque-2; et ester diéthylaminoéthylique de la carboxy-2-nitro-5-oxazolo-1,3-[4,5-h)quinoléine, qui consiste à faire réagir, dans des conditions de condensation, un composé de formule III:

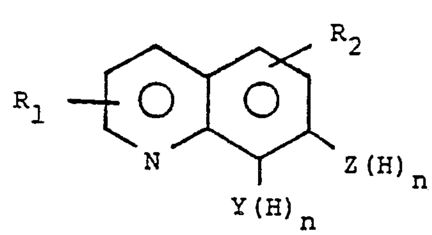

III

où:

$R_1$, $R_2$, Y et Z sont tels que définis ci-dessus; et

n vaut 1 ou 2,

avec un composé de formule IV:

X—R (IV)

où

X est tel que défini ci-dessus

R représente un groupe di- ou trifonctionnel capable de se condenser avec $Y(H)_n$ ou $Z(H)_n$ afin de former un noyau hétérocyclique; et facultativement, à former les N-oxydes correspondants du composé ainsi produit; et faculativement, à former des sels du composé ainsi produit.

2. Procedé selon la revendication 1, dans lequel m vaut 0.

3. Procedé selon la revendication 1 ou 2, dans lequel Z représente oxygène et Y représente azote.

4. Composé selon la revendication 1 ou 2, dans lequel Z représente soufre et Y représente soufre et Y représente azote.

5. Composé selon la revendication 1 ou 2, dans lequel Z représente azote et Y représente soufre.

6. Composé selon la revendication 1 ou 2, dans lequel Z représente azote et Y représente $R_3N$.

7. Procédé selon la revendication 1, dans lequel le produit formé est un composé carbalcoxy-2 qui, par saponification, donne le composé carboxy-2 correspondant.

8. Procédé selon la revendication 1, dans lequel le produit formé est un composé formyloximino-2- qui, par déshydration, donne le composé cyano-2-correspondant.

9. Procédé selon la revendication 1, dans lequel le composé cyano-2 est hydrolysé en le composé carboxy-2.

10. Procédé selon la revendication 1, dans lequel le composé cyano-2 est alcoolisé en le composé carbalcoxy-2.

11. Procédé de préparation d'une composition pharmaceutique, consistant à formuler une quantité thérapeutiquement efficace d'une composé préparé comme défini à l'une des revendications 1 à 10.